# EUROPEAN PATENT APPLICATION

(11) **EP 2 180 065 A1**
(43) Date of publication of application: **28.04.2010**
(21) Application number: 08167513.4
(22) Date of filing: 24.10.2008
(51) Int. Cl.: C12Q 1/68

(54) **Method of reducing the molecular weight of at least one PCR product for its detection while maintaining its identity**

(71) Applicant: Fraunhofer-Gesellschaft zur Förderung der angewandten Forschung e.V., 80686 München (DE)
(72) Inventor: Kirsten, Holger, 04318, Leipzig (DE); Ahnert, Peter, 04109, Leipzig (DE); Boltze, Johannes, 08439, Langenhessen (Werdau) (DE)
(74) Representative: Bühler, Dirk

(57) **Abstract**

The present invention relates to a method of detecting at least one specific PCR product wherein a specific signature sequence is introduced by primer design to each PCR product and, in further steps, the presence of said specific signature sequences is detected, e.g. by mass spectrometry.

## Description

### FIELD OF THE INVENTION

The present invention relates to the field of detecting at least one PCR product. This is achieved by introducing a specific signature sequence into each of said at least one PCR products followed by cleaving said at least one PCR product in order to provide said at least one specific signature sequence for detection.

In this respect, the method of the present invention comprises *inter alia* the provision of a DNA template, the provision of at least one forward primer comprising a specific signature sequence and the provision of at least one reverse primer. Said forward primer and said reverse primer hybridise to specific sequences at the 5' and 3' end of the sequence to be amplified.

Furthermore, the method of the present invention also comprises the provision of a DNA template, the provision of a "first" primer pair as mentioned above, wherein said two "first" primers comprise recognition elements for a second primer pair, and the provision of a second forward and a second reverse primer. Said second primer pair can be a universal primer pair.

### BACKGROUND OF THE INVENTION

The polymerase chain reaction (PCR) for the amplification of DNA sequences was discovered almost 25 years ago. Since then, the use of said reaction has dramatically influenced day-today work in laboratories. Today, the PCR method represents a widely used standard method in almost every laboratory with a broad range of application such as research, diagnostics or even business.

In the field of research, most of the cloning techniques, DNA "altering" methods such as site directed mutagenesis or the quantitative analysis of gene expression are largely depending on PCR techniques and related reverse transcription (RT)-PCR techniques. Furthermore, due to notable process over the last years, a wide variety of special PCR methods has been developed. Using elaborate conditions and polymerases with high proofreading and/or high processing activity, DNA sequences of up to 40 kb are routinely amplified today, e.g. for the cloning of large genes.

Furthermore, PCR techniques are widely used in the medical diagnostic field, e.g. to detect pathogens or to determine genotypes. Particularly with respect to the latter aspect, PCR methods have been developed as high throughput methods for the analysis of different PCR products at the same time. Since PCR products are at least in the initial reaction phase amplified proportional to the amount of template present, it is also possible to quantitatively analyse samples by PCR. Thus, standard high throughput PCR methods are carried out routinely today, e.g. to analyse genotypes such as SNPs in a human patient. In this respect, reference is made to the "GenoSNIP" method which is used as high throughput method in order to analyse SNPs by mass spectrometry (Bruker Daltonik GmbH, Wenzel T et al., (2003). Genosnip: SNP genotyping by MALDI-TOF MS using photocleavable oligonucleotides. Nucleosides Nucleotides Nucleic Acids. 22(5-8):1579-81.PMID: 14565470).

In this respect, it is crucial to not only develop and provide methods in order to amplify DNA sequences but also to provide methods which allow for specific detection of the corresponding PCR products in a way which is easy to handle, cost efficient and may be used in accordance with high throughput systems. As outlined above for the GenoSNIP method, the use of mass spectrometry for detection of PCR products has dramatically increased over the last years.

However, particularly the detection of longer PCR products still represents a problem for methods routinely used for the detection of PCR products. The use of mass spectrometry is e.g. limited to the detection of PCR products of specific sizes, i.e. detection with accuracy is possible today only for PCR products of up to about 250 nucleotides (Taranenko N et al., (1994). 3-Aminopicolinic acid as a matrix for laser desorption mass spectrometry of biopolymers. Rapid Commun Mass Spectrom. 12:1001-6.PMID: 7696697). Importantly, it should be noted that in a case where different larger PCR products are amplified, the resolution of the corresponding signals is compromised. Thus, in such a situation, the sizes of the different PCR products have to differ significantly in order to detect such products independently.

Thus, there is the need for a method which allows for the identification of a PCR product independent of its size. Furthermore, there is the need for a method which allows not only for the detection of one specific PCR product of any size but of more than one specific PCR product in a high throughput manner in case different PCR products are present in a sample.

### OBJECTS AND SUMMARY OF THE INVENTION

It is an objective of the present invention to provide a method that can be used for the detection of at least one specific PCR product of any length. Said method comprises *inter alia* the provision of a primer pair wherein each primer comprises specific sequences hybridising to the sequence to be amplified and wherein each forward primer comprises a specific signature sequence.

Furthermore, it is an objective of the present invention to provide a method that can be used for the detection of at least one specific PCR product comprising the provision of at least one first primer pair and a second primer pair, wherein the second primer pair may be universal.

According to said method of the present invention, both, the at least one first primer pair as well as the second primer pair can be used in a single PCR reaction.

Both objectives of the present invention may be used for detecting at least one specific PCR product in a sample.

These and other objectives of the present invention, as they will become apparent from the ensuing description, are solved by the subject matter of the independent claims. The dependent claims relate to some of the preferred embodiments of the invention.

According to one aspect of the invention, a method of detecting at least one specific PCR product is provided. Said method comprises at least the steps of:
a) providing a DNA template;
b) providing at least one forward primer wherein each forward primer comprises the following sequence elements in 5' to 3' direction:
   a. a specific signature sequence;
   b. a cleavable site B;
   c. a specific sequence hybridising to the DNA template at the 5' end of the sequence to be amplified;
c) providing at least one reverse primer wherein each reverse primer comprises in 5' to 3' direction a specific sequence hybridising to the DNA template at the 3' end of the sequence to be amplified;
d) performing a PCR reaction using the DNA template of step a) and the primers of steps b) and c);
e) cleaving DNA at said cleavable site to provide DNA cleavage products;
f) analysing DNA cleavage products for the presence of cleavage products comprising said at least one specific signature sequence and thus for the presence of the at least one specific PCR product.

In one embodiment of this first aspect the invention, each forward primer mentioned in step b) comprises an additional cleavable site A, such that the method comprises at least the steps of:
a) providing a DNA template;
b) providing at least one forward primer wherein each forward primer comprises the following sequence elements in 5' to 3' direction:
   a. a cleavable site A;
   b. a specific signature sequence;
   c. a cleavable site B;
   d. a specific sequence hybridising to the DNA template at the 5' end of the sequence to be amplified;
c) providing at least one reverse primer wherein each reverse primer comprises in 5' to 3' direction a specific sequence hybridising to the DNA template at the 3' end of the sequence to be amplified;
d) performing a PCR reaction using the DNA template of step a) and the primers of steps b) and c);
e) cleaving DNA at said cleavable sites to provide DNA cleavage products;
f) analysing DNA cleavage products for the presence of cleavage products comprising said at least one specific signature sequence and thus for the presence of the at least one specific PCR product.

In the first aspect of the invention, one may thus also refer to "an optional cleavable site A" comprised in each forward primer mentioned in step b) of a method.

In this first aspect of the invention, only one primer pair is used for each specific sequence to be amplified. Said primer pair is specific for the region to be amplified by comprising elements hybridising to the 5' and 3' ends of the sequence to be amplified. If two specific sequences should be amplified in one reaction, said first aspect of the invention comprises the provision of two first primer pairs wherein each primer pair comprises sequences specific for each specific sequence. If three specific sequences should be amplified, three specific primer pairs need to be provided according to the first aspect of the present invention, and so on.

According to the second aspect of the present invention, a further method of detecting at least one specific PCR product is provided. Said method comprises at least the steps of:
a) providing a DNA template;
b) providing at least one first forward primer wherein each first forward primer comprises the following sequence elements in 5' to 3' direction:
   a. a recognition element for a second forward primer;
   b. a specific signature sequence;
   c. a cleavable site B;
   d. a specific sequence hybridising to the DNA template at the 5' end of the sequence to be amplified;
c) providing at least one first reverse primer wherein each first reverse primer comprises in 5' to 3' direction:
   a. a recognition element for a second reverse primer;
   b. a specific sequence hybridising to the DNA template at the 3' end of the sequence to be amplified;
d) providing a second forward primer comprising in 5' to 3' direction said recognition element for the second forward primer and optionally a part of said signature sequence;
e) providing a second reverse primer comprising in 5' to 3' direction said recognition element for the second reverse primer;
f) performing a PCR reaction using the DNA template of step a) and the primers of steps b) to e);
g) cleaving DNA at said cleavable site to provide DNA cleavage products;
h) analysing DNA cleavage products for the presence of cleavage products comprising said at least one specific signature sequence and thus for the presence of the at least one specific PCR product.

In one embodiment of this second aspect the invention, each second forward primer mentioned in step d) comprises a cleavable site A, such that the method comprises at least the steps of:
a) providing a DNA template;
b) providing at least one first forward primer wherein each first forward primer comprises the following sequence elements in 5' to 3' direction:
   a. a recognition element for a second forward primer;
   b. a specific signature sequence;
   c. a cleavable site B;
   d. a specific sequence hybridising to the DNA template at the 5' end of the sequence to be amplified;
c) providing at least one first reverse primer wherein each first reverse primer comprises in 5' to 3' direction:
   a. a recognition element for a second reverse primer;
   b. a specific sequence hybridising to the DNA template at the 3' end of the sequence to be amplified;
d) providing a second forward primer comprising in 5' to 3' direction said recognition element for the second forward primer and optionally a part of said signature sequence wherein a cleavable site A is comprised in said recognition element or 5' to the recognition element;
e) providing a second reverse primer comprising in 5' to 3' direction said recognition element for the second reverse primer;
f) performing a PCR reaction using the DNA template of step a) and the primers of steps b) to e);
g) cleaving DNA at said cleavable sites to provide DNA cleavage products;
h) analysing DNA cleavage products for the presence of cleavage products comprising said at least one specific signature sequence and thus for the presence of the at least one specific PCR product.

In the second aspect of the invention, one may thus also refer to "an optional cleavable site A" comprised in each second forward primer mentioned in step d) of a method.

According to said second aspect of the present invention, at least one first primer pair and a universal second primer pair are provided. As in the first aspect of the invention mentioned above, said at least one first primer pair introduces the specificity for the DNA sequence to be amplified. However, both the forward and the reverse primer of the first primer pair also comprise recognition elements for a second primer pair. Said recognition elements may be identical in all of the at least one first primer pairs (in contrast to the specific sequences hybridising to the DNA template) and, thus, the second primer pair can be used with any of the first primer pair and may be referred to as being universal.

In preferred embodiments of both methods mentioned above, the analysis of the DNA cleavage products as outlined in step f) of the first aspect and in step h) of the second aspect is achieved by methods selected from the group of methods comprising mass spectrometry, gel electrophoresis and gel filtration.

If mass spectrometry is used as method for the analysis of the DNA cleavage products, it is preferred to use conventional MALDI-TOF technology.

In also preferred embodiments of both aspects of the present invention, the DNA cleavage products as mentioned above are purified prior to the analysis. Said purification may be performed by purification methods selected from the group of methods comprising gel filtration, DNA precipitation, desalting as well as chromatography, preferably affinity chromatography such as tag based systems or specific DNA binding devices. Said purification can also comprise the chemical modification of the DNA fragments resulting in detectable fragments.

The purification method chosen preferably depends on the cleavage method used; thus in case a digestion (either in parallel or sequential optionally including heat steps for inactivation) with two restriction enzymes is used, desalting and precipitation methods may be used for purification. However, if only one restriction enzyme is used in combination with a cut by UV-irradiation at a photocleavable linker and tagged DNA (e.g. biotin-tagged-DNA) the purification step can comprise a streptavidin-matrix in combination with washing steps.

Furthermore, in preferred embodiments of both aspects of the present invention, the sequence hybridising to the DNA template and/or the recognition element for the second forward primer and/or the recognition element for the second reverse primer comprises between 10 and 30 nucleotides with 20 nucleotides being preferred. However, in other embodiments, said sequence comprises between 6 and 8 nucleotides or between 40 and 100 nucleotides.

In further preferred embodiments of both aspects of the invention, the cleavable site A and/or the cleavable site B are cleavable by methods selected from the group of methods comprising a digestion with restriction endonucleases, UV light irradiation of photocleavable linkers and chemical cleavage reactions at specific nucleotide sequences.

In further preferred embodiments where both cleavage sites are present, both the cleavable site A and the cleavable site B are recognition sites for restriction endonucleases and cleavable by a digestion with restriction endonucleases.

Referring to the paragraph above, if the two cleavable sites are recognition sites for restriction endonucleases, it can, in both aspects of the present invention, be preferred that the cleavable site A and the cleavable site B are identical and cleavable by a digestion with one restriction endonuclease, preferably with DraI.

However, it can also be preferred that the cleavable site A and the cleavable site B are recognition sites for restriction endonucleases but differ in their sequence and are thus recognized by different restriction endonucleases and cleavable by a parallel digestion or a sequential digestion with two different restriction endonucleases, preferably with FseI and PmeI.

In other preferred embodiments of both aspects of the present invention where both cleavage sites are present, the cleavable site A comprises a photo-cleavable linker and is cleavable by UV light irradiation and the cleavable site B is a recognition site for a restriction endonuclease and cleavable by a digestion with a restriction endonuclease, preferably with DraI.

Preferred embodiments of the present invention can, furthermore, relate to methods wherein all of the at least one forward primers (with respect to the first aspect of the invention) or the second forward primer (with respect to the second aspect of the invention) are coupled at their 5' ends to either one tag of a tag pair selected from the group of tag pairs comprising streptavidin/biotin, hapten/anti-hapten, pairs of complementary nucleotide sequences, aptamers/aptamer targets, histidine glutathione-S-transferase/glutathione, 6X Histidine Tag/Ni-NTA, S- protein/S-peptide, Cutinase/phosphonate inhibitor, antigen/antibody, folic acid/folate binding protein, and protein A or G/immunoglobulins.

In particularly preferred embodiments relating to both aspects of the present invention, said tag is biotin tag. Thus, in the first aspect of the invention, the at least one forward primer is coupled at its 5' end to biotin, whereas in the second aspect of the present invention, the second forward primer is coupled at its 5' end to biotin.

The next embodiments of the present invention refer to a high throughput method with the goal of preferably detecting several different PCR products. One embodiment refers to the method using several different primer pairs without a second primer pair whereas the second embodiment refers to several different first primer pairs with an additional universal second primer pair. In a high throughput method, the second embodiment may be preferred. Both embodiments relate to methods where the two cleavable sites A and B are present. In both cases, the cleavable site B is a recognition site for a restriction endonuclease and is cleavable by a digestion with a restriction endonuclease, preferably with DraI, whereas the cleavable site A comprises a photo-cleavable linker and is cleavable by UV light irradiation. Furthermore, either the at least one forward primer or the second forward primer (depending on the method used, either according to aspect 1 or aspect 2) is coupled at its 5' end to a tag as outlined above. In both aspects, said embodiments relate to the cleavage of the DNA after performing a PCR reaction using the corresponding primers by a digestion with a restriction endonuclease cleaving at the cleavable site B, preferably with DraI. Subsequently, DNA fragments which comprise a tag as mentioned above are purified by appropriate purification methods (using said tags) and, following the purification and enrichment of tagged DNA fragments, said tagged DNA is then cleaved at cleavable site A by UV light irradiation.

Thus, in the preferred embodiments as set out in the last paragraph, cleavage products comprising said at least one specific signature sequence are provided in an enriched and ready-to-analyse situation. Therefore, said preferred embodiments may be referred to as high throughput method.

In the high throughput method of the preferred embodiments described above, the tag used for purification can be a biotin-tag and, thus, the biotin-tagged DNA can easily be purified via coupling to a streptavidin matrix followed by washing steps. In this regard, both aspects of the present invention are compatible to well known methods and kits used for high throughput screening, such as the GenoSNIP-method.

### DESCRIPTION OF THE FIGURES

In all figures, a general scheme is shown on the top. Said scheme may be adopted for any DNA target region to be amplified by PCR. If not explicitly mentioned, all DNA sequences are given in 5' to 3' direction. For further details, reference is made to the Example section of the present invention.

### Figure 1:

**Primer design of the first forward and the first reverse primer.** Bottom: As example for a sequence to be amplified, human genomic DNA around SNP rs708272 is given (SEQ IDs No. 1 and No. 2). The underlined base represents the site of the SNP (allelic variant G). Primer *P1for - specific for DNA variant A* (SEQ ID No. 3) comprises the sequence elements of the invention (*italic*: recognition element for second primer, **bold:** signature sequence, small letters: restriction site, capital letters: annealing sequence) with an A at the 3' end specific for genetic variant A. Primer *P1for - specific for DNA variant G* (SEQ ID No. 4) comprises a G at said position and, furthermore, the signature sequence differs from the signature sequence of the first primer. Finally, primer *P1for - specific for different DNA length* (SEQ ID No. 5) hybridises to a different 5' sequence of the genomic DNA and comprises a signature sequence different from the first two signature sequences. Primer *P1rev* (SEQ ID No. 6) may be identical in all three cases or may be different regarding its hybridising region. Primer *P1rev* does not necessarily comprise a recognition element for a second primer as it may be used with a second forward primer as well. Top: M1 indicates a signature sequence 1 and R indicates a recognition site for a restriction endonuclease; the resulting PCR product is also depicted.

### Figure 2:

**Primer Design of the second primer pair.** Bottom: The amplified DNA using the primer *P1for - specific for DNA variant G* and primer *P1rev* is given including the SNP (underlined G). The amplified region now also comprises at the 5' and 3' ends recognition sequences specific for the universal second primer pair (*italic*) wherein the primer *P2for* (SEQ ID No. 7) comprises a light-inducible cleavage site "L" as well as a biotin-tag at the 5' end. Note that Primer *P2rev* (SEQ ID No. 8) is optional. Top: Bio indicates a biotin-tag. In this scheme, the first reverse primer is used for the amplification; the resulting PCR product is also depicted.

### Figure 3:

**First cleavage reaction using a restriction endonuclease.** The biotin-tagged amplified DNA using the first and the second primer pairs is given with the recognition sequence for restriction endonuclease DraI in small letters. When cleaving at this site by a digestion with DraI, at least two cleavage fragments are obtained, wherein one comprises the signature sequence element as well as a biotin-tag at the 5' end. Biotin-tagged DNA fragments may thus be purified and enriched using the biotin-tag and a streptavidin matrix.

### Figure 4:

**Second cleavage reaction using UV-light irradiation.** The biotin-tagged DNA fragments (optionally still coupled to a streptavidin matrix) are now cleaved in a second cleavage step at the light-cleavable linker L by UV light irradiation. Corresponding fragments are shown on the right hand side. As final result, a cleavage product comprising the specific signature sequence introduced by primer *P1for - specific for DNA variant G* is present, wherein the molecular weight of said DNA cleavage product is specific for the PCR product since it is only present if the corresponding full length PCR product has successfully been amplified.

### Figure 5:

**Analysis of DNA cleavage fragments using MALDI-TOF.** In the detection step, the products obtained after the second cleavage reaction are analysed for the presence of the at least one specific cleavage fragment which comprises the signature sequence. In this step, not only the product of the expected molecular weight may be detected, but also unreacted primer *P2for.* However, the molecular weight of said primer is known and can be discriminated from the signal for the cleavage fragment comprising the specific signature sequence. Expected molecular weights of cleavage products comprising different signature sequences are also depicted to show that the masses are specific and can easily be assigned to the corresponding product, if present.

### Figure 6:

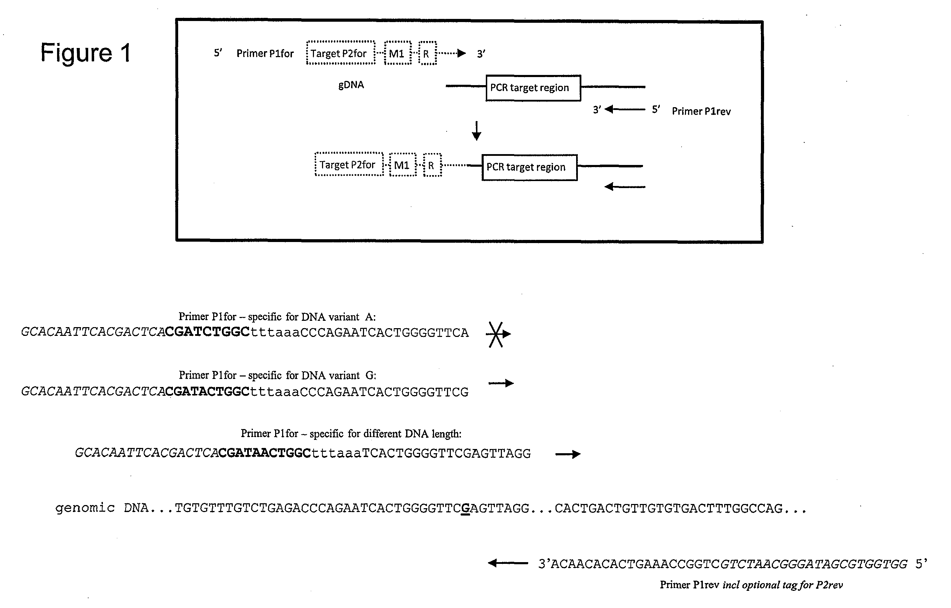

**Oligonucleotide ligation of primers OLA1 and OLA2.** The present invention may also be used with a ligation reaction of oligonucleotides as first step. Primer OLA1 is shown in the left, primer OLA2 in the right. OLA1 is comprised of the sequence elements as set out in figure 1. OLA2 comprises in addition to the hybridising region a recognition sequence for P2rev (in bold). Both primers anneal to the genomic DNA in close proximity such that a ligation reaction results in the a sequence as shown in the bottom of the figure. Using said product as template, a PCR with a second primer pair can be carried out.

### DETAILED DESCRIPTION OF THE INVENTION

The inventors have found that it is possible to detect at least one specific PCR product of any length by introducing a specific signature sequence into each of said PCR products followed by cleavage of said products in order to provide DNA cleavage products. Finally, a DNA cleavage product comprising said specific signature sequence is detected wherein each of the at least one specific signature sequence corresponds to each of the at least one specific PCR products.

In this respect, the inventors have found that only one primer pair may be used for each of the at least one specific PCR products. Furthermore, the inventors have found that it is also possible to use at least one first primer pair which is specific for a PCR product in combination with a universal second primer pair that may be used with all first primer pairs and in accordance with commercially available methods and kits, e.g. the GenoSNIP method.

The advantage of the present invention relates to the fact that it is possible to reduce the size of a PCR product of any length to a sequence known upfront, which is thus identifiable, by introducing said signature sequence during the PCR reaction. Using the method of the present invention, it is, in principle, possible to detect almost any specific PCR product, also if several specific PCR products are present in one sample. The specificity of the PCR reaction is, as in any regular PCR reaction, obtained by specific sequences in a primer pair used for the PCR reaction. Said sequences are designed in order to hybridise specifically to sequences of the DNA template such that the sequence to be amplified lies in-between said two primers. However, additional sequences present at the 5' ends of the primers flanking the sequence to be amplified may be introduced into the amplified sequence as well. The introduction of restriction sites during the process of cloning represents an example for said method. This can be achieved by designing the primers accordingly. In this regard, the inventors have found that it is possible to design primers in such a way that specific signature sequences are introduced to each of the sequences to be amplified.

Whilst describing in detail exemplary embodiments of the present invention, definitions important for understanding the present invention are provided.

As used in the specification and the appended claims, the singular form of "a" and "an" also includes the respective plurals, unless the context clearly dictates otherwise.

In the context of the present invention, the terms "about" and "approximately" denote an interval of accuracy that a person skilled in the art will understand to still ensure the technical effect of the feature in question. The term "typically" indicates a deviation from the indicated numerical value of ±10% and preferably ±5%.

It is to be understood that the term "comprising" is not limiting. For the purpose of the present invention, the term "consisting of" is considered to be a preferred embodiment of the term "comprising of". If, hereinafter, a group is defined as comprising at least a certain number of embodiments, this is also meant to encompass a group that preferably consists of these embodiments only.

As has been set out above, the present invention relates in one aspect to a method of detecting at least one specific PCR product. Said method comprises at least the steps of:
a) providing a DNA template;
b) providing at least one forward primer wherein each forward primer comprises the following sequence elements in 5' to 3' direction:
   a. an optional cleavable site A;
   b. a specific signature sequence;
   c. a cleavable site B;
   d. a specific sequence hybridising to the DNA template at the 5' end of the sequence to be amplified;
c) providing at least one reverse primer wherein each reverse primer comprises in 5' to 3' direction a specific sequence hybridising to the DNA template at the 3' end of the sequence to be amplified;
d) performing a PCR reaction using the DNA template of step a) and the primers of steps b) and c);
e) cleaving DNA at said cleavable site(s) to provide DNA cleavage products;
f) analysing DNA cleavage products for the presence of cleavage products comprising said at least one specific signature sequence and thus for the presence of the at least one specific PCR product.

Said aspect of the invention may preferably be used when only one specific PCR product is amplified from a DNA template. However, it is not limited to a PCR reaction of one product only. The new features of such a method as described above reside in the fact that the forward primer used during the PCR reaction is comprised of specific elements; as in any regular primer, a specific sequence hybridising to the DNA template at the 5' end of the sequence to be amplified is present. Of course, said sequence is located at the 3' end of the primer such that an elongation by a PCR polymerase can proceed in 3' direction. However, in addition to said sequence, a specific signature sequence is also present in said primer; said specific signature sequence is, furthermore, flanked by a cleavable site B and an optional cleavable site A such that after cleaving a specific DNA cleavage product comprising the specific signature sequence is provided.

As also already set out above, the present invention relates in another aspect to a method of detecting at least one specific PCR product. In said second aspect of the invention, the method comprises at least the steps of:
a) providing a DNA template;
b) providing at least one first forward primer wherein each first forward primer comprises the following sequence elements in 5' to 3' direction:
   a. a recognition element for a second forward primer;
   b. a specific signature sequence;
   c. a cleavable site B;
   d. a specific sequence hybridising to the DNA template at the 5' end of the sequence to be amplified;
c) providing at least one first reverse primer wherein each first reverse primer comprises in 5' to 3' direction:
   a. a recognition element for a second reverse primer;
   b. a specific sequence hybridising to the DNA template at the 3' end of the sequence to be amplified;
d) providing a second forward primer comprising in 5' to 3' direction said recognition element for the second forward primer and optionally a part of said signature sequence wherein an optional cleavable site A is comprised in said recognition element or 5' to the recognition element;
e) providing a second reverse primer comprising in 5' to 3' direction said recognition element for the second reverse primer;
f) performing a PCR reaction using the DNA template of step a) and the primers of steps b) to e);
g) cleaving DNA at said cleavable site(s) to provide DNA cleavage products;
h) analysing DNA cleavage products for the presence of cleavage products comprising said at least one specific signature sequence and thus for the presence of the at least one specific PCR product.

Said second aspect of the invention preferably relates to a method which can be used for the detection of several PCR products in a high throughput manner. The specificity of the resulting amplified PCR products is obtained by designing corresponding first forward and reverse primers, wherein each forward primer of said first forward primers (if more than one first primer pair is used) comprises a specific signature sequence such that the correlation of said specific signature to the corresponding PCR product is possible and unambiguous. Only if the first primer pair leads to a PCR product, the universal second primer pair is able to hybridise to the 5' and 3' ends of said PCR product. This is due to the fact that recognition elements for the forward and the reverse primers of the second primer pair are specifically introduced by the first primer pair. This also represents a control step such that the PCR reaction using said first primer pair was successful. If a reaction using a DNA template and a first primer pair was unsuccessful, the second primer pair would not lead to a product comprising the complete signature sequence. Said control step is, however, not the only advantage of using a second primer pair. If a second primer pair according to the features set out above is used, i.e. a universal second primer pair, it is, furthermore, possible by introducing a tag to the second forward primer at the 5' end to specifically enrich reaction products following the amplification reaction. In order to enrich said PCR products, only the universal second primer needs to be tagged and not every first forward primer (wherein said forward primers differ to introduce specificity to the reaction in contrast to the universal second forward primer). Said feature also introduces the advantage of a method of the present invention to be compatible with already known and routinely used detection systems for tagged DNA fragments, such as the GenoSNIP method.

In the following, general terms as used in the description of the present invention will be explained and defined.

The term "PCR product" as used herein refers to a DNA sequence, which has been amplified in a PCR reaction using primers flanking said sequence at the 5' and 3' ends. In the following, this will be explained in a simplified and schematic view. A linear DNA template may be comprised of a leading strand (in 5' to 3' direction, in the meaning of a "coding" strand) of 1,000 nucleotides and the corresponding complementary lagging strand, resulting in a DNA double strand. The goal may be the amplification of a sequence comprising nucleotides 10 to 520. A forward primer may thus comprise the nucleotides 10 to 30 of the leading strand in 5' to 3' direction, wherein said nucleotides are complementary to the corresponding nucleotides on the lagging strand and thus hybridise to said region. As they are directed in 3' direction towards nucleotide 1000, a polymerase will amplify the sequence of the lagging strand using said primer as priming site towards nucleotide 1000. In addition to said forward primer, a reverse primer is also present. Said reverse primer may comprise in 5' to 3' direction the complementary sequence of bases 520 to 500 of the leading strand. Thus, the nucleotides are complementary to the leading strand and will anneal to said strand. A corresponding PCR reaction will thus result in the extension of said reverse primer towards the 5' end of the leading strand, i.e. towards nucleotide 1. In the present example, the specific PCR product is a product ranging from nucleotide 10 to nucleotide 520 of the DNA template. The specificity for a PCR reaction resulting in a specific PCR product is thus introduced by the sequences of the primers which are annealing to the leading and lagging strands of the double stranded DNA template.

The term "complementary" is well-known to the person skilled in the art as it relates to the basic properties of nucleic acid molecules hybridizing with each other in view of the ability of adenosine to pair with thymine and uracil and guanidine to pair with cytidine.

The PCR product may furthermore comprise any artificially introduced sequences at both ends which are present in the two primers at the very 5' ends (said introduced sequences will of course not anneal to the template). In general, the length of such sequences seems to be limited by the methods used for primer synthesis only.

The term "specific" in the context of a PCR product therefore refers to a product of a specific sequence with respect to the length and the base sequence, the primers are annealing to. If, in the above example, the forward primer would comprise nucleotides 25 to 45 of the leading strand, the PCR product would then comprise nucleotides 25 to 520 and thus be different from the first product. Said difference may be resolved by the method of the present invention. However, it needs to be understood that the sequence in-between said two specific primers cannot be resolved by the present invention. If said sequence in-between comprises different bases (e.g. a SNP), this may not be resolved by using an identical primer pair. However, such a situation may be solved by designing different primers which are directly adjacent to said differing base and thus allow for amplification of the product only if the last base at the 3' end is annealing. This will be outlined in detail in the example section.

In summary, the term "specific PCR product" thus relates to a specific PCR product, wherein the specificity is provided by designing the forward as well as the reverse primer such that primer sequences anneal to specific sequences of the DNA. Said primer sequences are also referred to as "specific sequences hybridising to the DNA template" in the present invention.

The term "DNA template" is used herein as known to the skilled person. For a regular PCR reaction, a DNA template comprising two complimentary DNA strands, i.e. one leading and one lagging strand, needs to be present. Alternatively, a single DNA strand may also be used, such as in a "oligonucleotide ligation reaction" system as outlined below. Said DNA template used according to the present invention is provided outside the human or animal body. It may, however, be not only of human origin, but also of animal origin or derived from organism such as bacteriae, plants, fungi or archeae or produced by chemical synthesis methods and thus be of synthetic origin. It is preferred that for a diagnostic high throughput screening, the DNA template is derived from a human patient. Any method for isolating DNA from a human patient known to the skilled person may be used; in the context of research methods, the DNA template may also be a vector used for cloning and/or shuffling of genes and the like.

As already explained above, a "forward primer" represents a primer used for amplification processes during a PCR reaction. The forward primer is always designed in complementary fashion to the reverse primer such that the amplification directions are opposite and the corresponding two reaction products can anneal in a next step during the PCR reaction. In the example outlined above, the forward primer is e.g. complementary to the lagging strand. However, it may also be complementary to the leading strand if the reverse primer is designed accordingly. A forward primer is usually designed at the 5' end of the sequence to be amplified and thus flanks the 5'end of the PCR product.

Correspondingly, a "reverse primer" is comprised of nucleotides on the complementary strand of the forward primer. The reverse primer will flank the PCR product at the 3' end and, thus, it is designed such that its 5'end starts with the last base of the sequence to be amplified with the direction in 3' towards the 5' end of the sequence to be amplified. The sequence amplified using the reverse primer will thus be complimentary to the sequence of the reaction using the forward primer.

As pointed out above, a DNA template may be comprised of a leading strand (in 5' to 3' direction) and the corresponding reverse complementary strand (also referred to as lagging strand). With the definitions as given above for the primers, a forward primer thus hybridizes to the complementary strand of the DNA template at the 5' end and said reverse primer hybridizes to the leading strand (i.e. the strand in 5' to 3' direction) of the DNA template at the 3' end of the sequence to be amplified such that said primers hybridize to complementary strands of the DNA and are directed in 5' to 3' direction towards each other. In the second object of the present invention, this applies to the first forward primer and the first reverse primer, respectively.

The term "cleavable site" refers to either a sequence or a modified nucleotide or any further modification, which can be introduced by a PCR reaction and/or is compatible with a PCR reaction and at which and/or around which cleavage of the double stranded DNA can be initiated.

Most prominent examples for cleavable sites are recognition sites for restriction endonucleases. Said sites may thus be referred to as cleavable by enzymes. Typically, said recognition sites are comprised of about 4 to about 8 nucleotides, often in a palindromic manner, serving as docking sites for restriction endonucleases. Depending on the type of restriction endonuclease, either blunt ends or ends comprising overhangs will be present after the cleavage reaction. The recognition sequence may be any recognition site known for restriction endonucleases. However, it may be preferred that rare sequences for "rare" restriction endonucleases are used. The use of DraI may be particularly preferred.

If both cleavable sites A and B are present, cleavable site A and cleavable site B may be identical and recognised by the same restriction endonuclease. However, they may also differ and thus be recognised by two different enzymes. This may in some embodiments allow for a sequential cleavage of the amplified sequences which may be advantageous with respect to the purification. Thus, a first purification step may be introduced after the first cleavage reaction. It can be preferred that said two corresponding recognition sites are sites recognised by FseI and PmeI.

Said cleavable site can also comprise a modified base or a cleavable linker introduced during primer synthesis which are cleavage by e.g. UV-light, such as 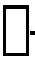 -cyanoethylphosphoramidite.
Of course, in this case, the cleavable site preferably needs to be introduced via the corresponding primer, since it is not easily possible to introduce a photocleavable base via a regular PCR reaction (e.g. via the addition as modified nucleotide as this could result in uncontrolled incorporation of said modified nucleotide). However, when comprised in a primer, such a photocleavable site is compatible with a PCR reaction, since it does not disturb the annealing process of the primer to the DNA template to a degree, that specificity of said annealing process would be lost. Furthermore, it is part of the PCR product since it resides in the primer sequence used as priming site and thus is part of the product.

According to the present invention, the use of cleavable sites recognised by restriction endonucleases, or of cleavable sites carrying photo-cleavable modified bases may be preferred; however, any other PCR-compatible method may be used as well. This may comprise sequences which are recognised by certain chemicals resulting in a cut at the corresponding position.

The optional cleavable site A and cleavable site B can independently be chosen and combined from any of the possibilities as set out above.

However, one may also use physical and/or chemical cleavage methods which cleave at unspecific sites within the amplified sequence in such a way that the sizes of the resulting DNA fragments are specific and can be predicted. Thus, again, the expected mass of a fragment comprising a specific signature sequence is known, and the sample may be analysed for said mass. However, cleavage methods comprising cleavage at the introduced cleavage site(s) as outlined above are preferred for a method according to the present invention.

The term "specific signature sequence" as used in the present invention refers to a sequence of defined bases of a known molecular weight. The term "specific" as used in the expression refers to the fact that for each specific PCR product, a specific signature sequence is present in the corresponding forward primer. Only if the corresponding DNA sequence is amplified and the PCR product is present, the specific signature sequence for said PCR product will also be present. If two PCR products are amplified using two different primer sets or two different forward primers with a common reverse primer, two specific signature sequences differing from each other will be present in said two forward primers and thus in the products. If three PCR products are amplified using three different primer sets or three different forward primers with a common reverse primer, three specific signature sequences differing from each other will be present in said three forward primers and thus in the products, and so on.

The specific signature sequence is preferably a short sequence. The term "short" may relate to a sequence of about 4 to about 50 nucleotides, preferably to about 5 to about 30 nucleotides, more preferably to about 6 to about 20 nucleotides and most preferably to about 7 to about 15 nucleotides. In principle, however, there is no limitation to the size and composition of the signature sequence; thus, said sequence may comprise from about 2 to about 250 nucleotides. however, when using methods such as MALDI-TOF or mass spectrometry in general, it can be preferred that the signature sequences do not exceed the length of about 250 nucleotides, more preferably that said sequences do not exceed the length of about 50 nucleotides.

If more than one signature sequence is used, said signature sequences may differ in that for each further signature sequence, a further base may be introduced into the sequence; thus for the first signature sequence, 10 nucleotides of a known composition may be used, whereas in the second signature sequence, 11 nucleotides of a known composition may be used wherein 10 correspond to the 10 of the first signature sequence with one additional base, accounting for a differentiation between said two signature sequences. In general, there are almost indefinite possibilities and varieties of designing corresponding signature sequences. The only limitation is, however, that it has to be excluded that two different signature sequences result in a similar signal in the detection step.

Thus, the method according to the present invention can preferably comprise an additional step of making sure that the molecular weights of the signature sequences are different if more than one is used. If e.g. two sequences are of identical length and composition except for one base, their molecular weights differ since the four nucleotides A, T, G and C (also referred to as "bases" herein) have different masses as shown below:

| Nucleotide | mass in D |
|---|---|
| A | 313.2 |
| C | 289.2 |
| G | 329.2 |
| T | 304.2 |

The skilled person known that there are also software tools in order to design signature sequences with the properties as set out above. Said tools often allow for the calculation and determination of the molecular mass of the corresponding DNA cleavage product comprising said signature sequence. Examples for software are Calcdalton (Kirsten H et al.,(2006). CalcDalton: a tool for multiplex genotyping primer design for single-base extension reactions using cleavable primers. Biotechniques 40:158-62.PMID: 16526404), Netprimer (http://www.premierbiosoft.com/jsp/marketing/FreeToolLogin.jsp?PID=3), and Genotools (Pusch W et al.,(2001). Genotools SNP manager: a new software for automated high-throughput MALDI-TOF mass spectrometry SNP genotyping. Biotechniques 30:210-5.PMID: 11196313).

Any modified nucleotides which can be used in accordance with a PCR reaction (and are thus amplifiable) and which may be introduced into the primer via the corresponding synthesis may as well be used. Of course, their molecular weight is known as well and can be calculated into the resulting final mass of the fragment. Examples for modified nucleotide applicable in PCR reactions which may be used in a signature sequence are thiophosphate-modified nucleotides (2'-deoxynucleoside 5'-□-[P-thio]-triphposphates) or boranophosphates (2'-deoxynucleoside 5'-□-[P-borano]-triphosphates).

As set out above, the DNA cleavage products are in preferred embodiments analysed via mass spectrometry, e.g. MALDI-TOF. In such an analysis, it is possible to distinguish between two samples comprising signature sequences which differ in approximately 0.5% in their molecular masses. This again shows how sensitive said detection is. Furthermore, it is obvious, how many possibilities and varieties there are to design corresponding signature sequences.

It needs to be understood that the molecular weight of the signature sequence does not necessarily reflect the molecular weight of cleavage product analysed in the last step of a method of the present invention. Due to the at least one cleavable site flanking the signature sequence, additional bases may be additionally comprised in the cleavage product (e.g. due to remaining bases after cleavage using restriction endonucleases since many of said restriction endonucleases cut in the middle of a recognition sequence). However, as the cleavable site(s) is/are designed identically in all primers in all forward primers comprising different signature sequences, all remaining bases will be identical in the cleavage products.

The term "PCR reaction" is used as known to the skilled person. Briefly, a PCR reaction comprises at least the following steps:

A DNA template and at least one forward as well as at least one reverse primer as set out above are provided in a reaction tube. Additionally, a DNA polymerase, the corresponding dNTP's and corresponding buffer conditions (such as salt concentrations and the like) are provided in said tube. In the initial denaturing step at a temperature of about 95°C of up to several minutes, the double stranded DNA template is melting and one leading and one lagging strand are therefore present in the sample. During the next step wherein the temperature is lowered to an annealing temperature and held for several seconds up to about 1 minute, the two primers anneal to the corresponding strands of the DNA template, i.e. one to the leading and the other primer to the lagging strand. Typical annealing temperatures range from about 40 to about 65°C. In the next, step, the elongation step, the temperature is increased to the optimal working temperature for the polymerase used (about 65 to about 75°C), such that the polymerase elongates the primers using the complementary strand as template. Depending on the polymerase, certain incubation times are chosen for this step, e.g. 2 minutes per 1 kb to be amplified. Finally, in the last step, the temperature is again increased to a denaturing temperature such that the double stranded products of the first elongation reaction are separated. Following this denaturation step, the next cycle comprising annealing, elongation and denaturing is then initiated. Typically, PCR reactions comprise about 20 to about 40 cycles. Depending on the purpose of the PCR reaction, the polymerases chosen for the reaction as well as the buffer conditions and the incubation cycles can differ to a large extent. However, said setups are known to the skilled person. The present invention may be used with any PCR reaction and is not limited to certain reactions only.

It is important to note that a PCR reaction according to the second aspect of the invention, i.e. a reaction wherein several primer pairs are present comprising also a universal second primer pair, does not comprise further steps. The amplification reactions using the different primer pairs can be done simultaneously.

In this regard it should further be noted that an amplification reaction may also take place using a template, which has been amplified using a first primer pair (thus comprising recognition sequences for the second forward primer), the second forward primer annealing to the 5' end and the first reverse primer annealing to the 3' end. However, it is obvious that such an amplification is still specific for the specific PCR product since it will produce the full length product comprising the specific signature sequence. A reaction using the first forward primer and the first and/or second reverse primer, however, will not result in a detectable PCR product since it will lack the properties introduced by the second forward primer.

It should, furthermore, be noted that the at least two different primer pairs may be added in different amounts (or to different end concentrations, respectively) in such a way, that the amount of the universal second primer pair is larger than the amount(s) of the at least one first primer pair. By adjusting the final concentrations accordingly, it is thus possible to direct the amplification reaction to a process using preferably the second primer pair, once the products using the at least one first primer pair reach a critical amount.

The term "cleaving DNA" as used herein means at least one cleavage reaction at the two cleavage sites A and/or B present in the DNA products after performing a PCR reaction. However, cleaving DNA does not exclude the possibility that the PCR products as well as the DNA template is cleaved in other regions as well with the exclusion that the specific signature sequence is cleaved other than at sites A and/or B. According to the present invention, the signature sequence itself must be a sequence which is not cleavable by a cleavage reaction used according to the present invention, but is flanked only by optionally two of such cleavage sites. As the method of the present invention is directed to the detection and identification of PCR products, the corresponding remaining at least one PCR cleavage product itself does not need to be present anymore. Thus, it may also be cleaved at other sites. However, the method of the present invention may also be used with cleavable sites which are not present in the DNA template (and thus not in the PCR products). Such a set up may be advantageous for applications wherein purification and enrichment steps rely on the length of the corresponding DNA fragments (such as e.g. gel filtration).

In the last step of the objectives of the present invention, the DNA cleavage products are "analysed for the presence of cleavage products comprising said at least one specific signature sequence". As mentioned above, not only the signature sequence itself may be detected, but a cleavage product which may comprise additional bases than the signature sequence due to cleavage reactions. Said analysis may be performed using a method selected from the group of methods comprising mass spectrometry, gel electrophoresis and gel filtration. If mass spectrometry is used, the corresponding molecular masses of the cleavage products are analysed.

In general, it is important to note that any technique may be used, which allows for the discrimination between different sequences comprising nucleotides of preferably short lengths. By applying the method of the present invention, it is possible to reduce a PCR product of any size down to an identifiable and known DNA cleavage product comprising a known signature sequence and, thus, the methods used for the analysis are not limited to the analysis of small PCR products only. Methods allowing for high throughput screening such as mass spectrometry are most preferred methods for said analysis.

If gel electrophoresis methods are used, they may comprise a native or a denaturating gel electrophoresis. Detection steps used in electrophoresis and/or filtration methods may rely on the detection of the DNA cleavage product comprising the signature sequence by UV- or fluorescence detection systems due to the addition of specific dyes, e.g. DNA-intercalating dyes. Such systems are known to the skilled person. Alternatively, modified nucleotides comprising e.g. fluorescent dyes may already be present in the universal second forward primer and thus be detected in combination with detection of the expected size of the DNA cleavage products. In preferred embodiments of the invention where methods as outlined in this paragraph are used for the detection of DNA fragments comprising signature sequences, it is preferred that the optional cleavable site A is absent since there is no need for an additional enrichment and/or purification step using a tag followed by the cleavage of the DNA fragment from said tag.

The term "recognition element" as used herein describes a sequence which is in one aspect of the invention present in the at least one first primer pair. Said recognition elements are identical if more than one first primer pair is used, i.e. in all first forward primers a specific recognition element is present and in all first reverse primers a specific recognition element is present (however, differing from the recognition element in the forward primers). Said recognition element may comprise up to 100 bases, preferably said element may comprise about 40, about 30, about 25 or more preferably about 20 bases.

Thus, a universal second primer pair can be used wherein the forward primer of the second primer pair recognises the complementary 5' recognition element in the corresponding PCR product (and, thus, comprises the identical recognition element as the first forward primer) and wherein the second reverse primer comprises a recognition element which hybridises to the complementary strand of the PCR product and is identical to the recognition element of the first reverse primer.

The second forward primer may be coupled to biotin at its 5' end such that the products of the PCR reaction may be purified via said biotin-tag. As outlined in detail below, this feature will enable the present invention to be compatible with kits which are already on the market and easy to use. Thus, the present invention may easily be integrated into already existing high throughput platforms.

As outlined above, the second forward primer comprises in 5' to 3' direction said recognition element and optionally a part of said signature sequence. It needs to be understood that the second forward primer does, however, in no case comprise the total signature sequence since in this case, discrimination between the different signature sequences would no longer be possible. However, the second primer may comprise partly said sequence, i.e. overlap to about 95%, to about 90%, to about 80%, to about 70%, to about 60%, to about 50%, to about 40%, to about 30%, to about 20% or to about 10% with said signature sequence such that remaining bases are still different and specific in the remaining sequence of the signature sequence. However, the second forward primer may not comprise any signature sequence as well. If many PCR products are to be detected, it is preferred that the second forward primer only comprises the recognition element without any parts of the signature sequences.

Furthermore, said second forward primer optionally comprises a cleavable site A. In the following, embodiments are discussed, wherein said site is present. Said second forward primer may comprises a cleavable site A either within said recognition element or 5' to the recognition element. This means that the second cleavable site A would, if it is present in the recognition element and amplifiable in a PCR reaction (such as a site recognised by restriction endonucleases) also be present in the first forward primer. If it is a photocleavable site, however, it is obvious that it would not be present in the first forward primer. However, said cleavable site A may also be present on the second forward primer in a region 5' to the recognition element and thus outside the sequence of the first forward primer. If the cleavable site A is indeed located upstream to the recognition element, it is obvious to the skilled person that the specific signature sequence will be accompanied by the complete recognition element of the first forward primer in a DNA cleavage product.

Prior to the analysis by any of the methods mentioned above, it may be necessary and preferred to purify the resulting DNA cleavage products. If tagged DNA fragments are chosen for further analysis, said fragments may be purified and enriched using specific methods depending on the tag. Said technique allows for high throughput screening and may even be used in an automated way. If e.g. two restriction endonucleases are used for cleavage, any purification method known to the skilled person, such as desalting methods or precipitation techniques of DNA fragments can be used. Size exclusion (e.g. gel filtration) may also be an appropriate method since the size of the resulting DNA fragments to be analysed is known from the beginning and, thus, a corresponding method may be used and optimised in order to purify and enrich corresponding cleavage products. In this regards, chromatography methods such as agarose or sephadex^{™} gels or columns may be used as well as affinity chromatography methods comprising DNA binding membranes or beads and the like.

A preferred embodiment of the first aspect of the present invention relates to a tag at each of the at least one forward primer, which also comprises elements introducing the specificity of the corresponding PCR reaction. In this regard, it needs to be understood that if more than one first forward primer is used, each of the forward primers comprising different sequences needs to be tagged at the 5' end, e.g. with a biotin tag.

With regard to the second aspect of the present invention, said universal second primer pair may be used for tagging, preferably the second forward primer. As the second primer pair is universal, there is no need to tag different forward primers with a biotin tag but use only the second forward primer for the tagging reaction with biotin. In this way, an easy and reliable high throughput procedure is possible.

Appropriate tags are selected from either one tag of a tag pair selected from the group of tag pairs comprising streptavidin/biotin, hapten/anti-hapten, pairs of complementary nucleotide sequences, aptamers/aptamer targets, histidine glutathione-S-transferase/glutathione, 6X Histidine Tag/Ni-NTA, S- protein/S-peptide, Cutinase/phosphonate inhibitor, antigen/antibody, folic acid/folate binding protein, and protein A or G/immunoglobulins.

If in either case at least one tagged forward primer is used, it is preferred that the cleavage site B is a sequence recognised by restriction endonucleases and cut by said enzymes. After the PCR reaction has been completed, the amplified DNA sequences may then be cut in the following step by a digestion with said restriction endonuclease. This will result in a fragment carrying at the 5' end a tag, such as biotin, and comprising the signature sequence.

Using said tag, it is possible to specifically enrich said fragments and thus purify said fragments by simply carrying out washing steps using wash buffer.

After the tagged sequences have been enriched (e.g. for biotin, a corresponding streptavidin matrix is provided for binding), said fragments are cleaved at cleavable site A, if present; it is preferred that said cleavable site A is under these circumstances a site comprising a photo cleavable linker and thus, UV irradiation is used to cleave at the cleavable site A. This cut will result in a sequence carrying the tag (optionally still coupled to a matrix) and a cleavage product comprising the signature sequence. It is evident that the molecular weight of the signature sequence, or if more than one signature sequence is used, the molecular weight for each of the specific signature sequences is known. Therefore, the fragments are analysed for the expected molecular weights and may then be easily assigned to the corresponding PCR product.

Alternatively, if the cleavable site A is not present, the binding between the two tags of the tag pair, i.e. in the example above biotin and streptavidin, may be broken, e.g. using water at elevated temperatures. This would also result in specific DNA fragments comprising the signature sequence.

The preferred embodiment described above represents a method which is compatible to already existing kits and methods, such as the GenoSNIP method. It may thus be easily used with a platform adapted to said system. Overall, this results in a method which allows for a high throughput screening using simple PCR primers wherein a specific signature sequence is introduced by said primers to each of the specific PCR products.

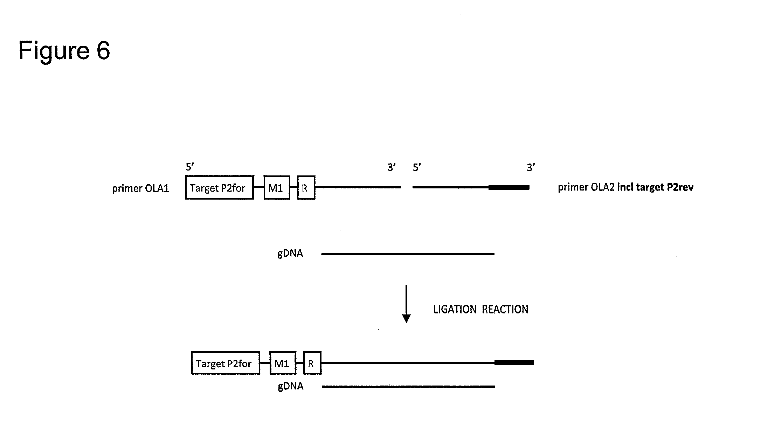

It should be pointed out that the present invention may also be used when a so-called "oligonucleotide ligation reaction" is carried out. In such a reaction, two primers as depicted in Figure 6 may be used. The primer OLA1, which represents the forward primer for the ligation reaction, is identical to either the forward primer described in the first objective of the present invention or identical to the first forward primer described in the second objective in the present invention. However, the reverse primer OLA2 is different from the reverse primers as described above: it is comprised of a sequence in close proximity, i.e. it hybridises immediately to the 3' side of the first forward primer, carries in addition a phosphate at its 5' site and hybridises to the identical strand as the forward primer. Thus, both of them either target the leading or the lagging strand. At the 3' end, the first reverse primer may comprise a recognition element for the second reverse primer P2rev. Only if said two primers anneal to the sequence of interest in the DNA template in direct proximity, the corresponding ligation reaction will take place and one "large primer" consisting of ligated primers OLA1 and OLA2 will be present as one strand at the DNA template.

As in the methods described above, the second reaction will then be a PCR amplification reaction. In this amplification reaction, a second forward primer comprising the sequence of the recognition site in OLA1 in 5' to 3' direction towards OLA2 and a second reverse primer comprising the complementary sequence to the recognition site in OLA2 in 5' to 3' direction towards OLA1 may be present. The remaining steps of the method are identical to the methods as described above. It is obvious to the skilled person that in such a system the initial DNA template comprises single stranded DNA only. Thus, it is also obvious to the skilled person that any definitions as given above with respect to a "PCR reaction", a "PCR product", a "DNA template", a "forward" and a "reverse" primer and the corresponding hybridisation reactions of course can be adjusted and thus also apply to a system, wherein the template is single stranded DNA only. In such as system, e.g. primer hybridisation would take place initially on one strand only.

In the embodiment described above, wherein a ligation reaction underlines the corresponding PCR reaction, the overall applicability of the method of the present invention becomes clear; thus, any primer based reaction comprising also a step of amplification by PCR may be used in accordance with the present invention in order to reduce the molecular weight of a PCR product or the product of a ligation reaction or the like of any length to a specific sequence comprising a signature sequence which may easily be detected.

The method of the present invention may be used for detecting at least one specific PCR product in a sample. A possible area of application thus relates to the detection of pathogens in a subject suspicious of e.g. an infection or the like. For this purpose, a DNA sample of said subject is provided outside the human or animal body. Said DNA is then analysed with a first first primer pair (comprising signature sequence A) specific for the subject's DNA region and a second first primer pair (comprising signature sequence B) specific for a DNA region of the pathogen. Together with a universal primer pair as set out above, the PCR reaction is carried out followed by cleavage of the amplified sequences. Finally, the DNA fragments are analysed for cleavage products comprising signature sequence A and for cleavage products comprising signature sequence B indicating the presence of the pathogen.

In the following, examples of the methods of the present invention are outlined. Said examples should, however, not be construed as limiting the scope of the present invention.

### EXAMPLES

### Example 1: Allele specific PCR for the detection of polymorphism G279A (rs708272)

The goal of this example is to determine the genotype of single nucleotide polymorphism (SNP) rs708272 of a human subject. Therefore, genomic DNA of the subject is provided outside the human or animal body. Said genomic DNA is preferably purified according to standard purification methods. A subject may be heterozygous at rs708272 corresponding to a G at the respective position on one allele and an A on the second allele. However, a subject may also be homozygous either for G or A at the respective position corresponding to two identical alleles.

As shown in Figure 1, two specific forward primers are used in the PCR reaction in order to determine the type of polymorphism.

Primer *P1for - specific for DNA variant A* is specific for DNA variant A. As depicted in Figure 1, said primer comprises in 5' to 3' direction a recognition element for a second forward primer followed by a specific signature sequence (in bold) and a cleavable site (in small letters), which corresponds to the sequence recognised by the restriction endonuclease DraI, i.e. TTTAAA. Finally, at the 3' end, said primer comprises the region which is specific for the DNA template, ending with an A at the very last 3' position such that a polymerase will only amplify from the 3' end if a T is present on the corresponding DNA template strand (i.e. said primer is specific for the allele with an A at position rs708272).

Primer *P1for - specific for DNA variant G* is also present in the PCR reaction. Said primer differs in two aspects from the first forward primer described above: the signature sequence differs from the signature sequence of the first primer in that an additional A is present in the sequence and the primer ends with a G as the very last base at the 3' end. Thus, a PCR product will be generated only if a C is present on the corresponding template strand at said position (i.e. said primer is specific for the second variant, namely a G at position rs708272).

For both possible reactions, a single reverse primer is sufficient, wherein said reverse primer comprises in 5' to 3' direction a recognition sequence for a second reverse primer as well as the sequence hybridising to the 3' end of the sequence to be amplified (see *P1rev* in Figure 1). It should be noted that the tag for a second reverse primer is optional since the first reverse primer can be sufficient for the second amplification reaction with the second forward primer.

In the present example, the genomic DNA analysed comprises two alleles with a G at rs708272. Thus, the subject is homozygous for genotype variant G at rs708272.

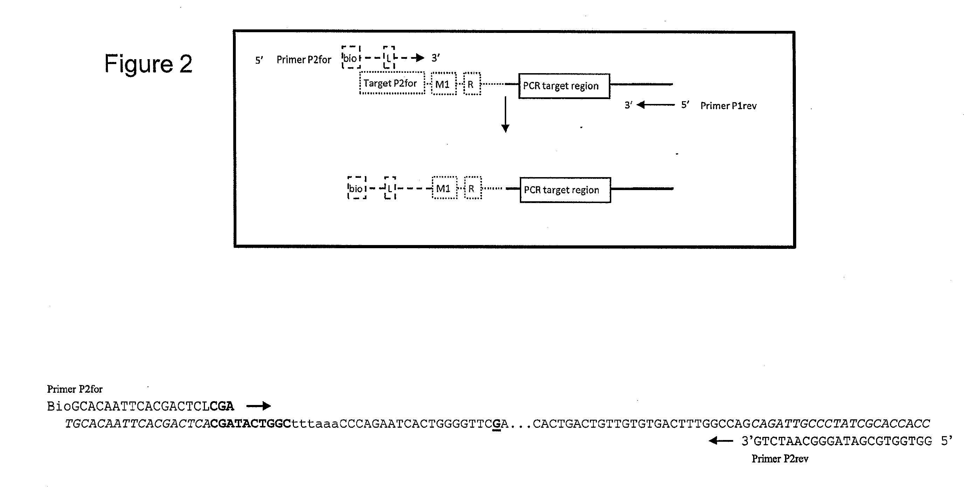

Accordingly, primer *P1for - specific for variant A* will not function as priming site for a polymerase and no PCR product will be generated using said primer as indicated in figure 1. However, primer P1for - *specific for variant G* will lead to PCR products and its sequence will be part of the amplified sequences. Said amplified sequence is shown in figure 2. Of course, additional sequence of primer *P1rev* (such as a recognition element for a second reverse primer) is also comprised in the amplified sequence, see figure 2.

The PCR reaction comprises a further primer pair: A second primer pair as depicted in figure 2 is also present. In the example shown here, the second reverse primer *P2rev* comprises the region of the recognition element of the first reverse primer only whereas the second forward primer *P2for* comprises a recognition element, an additional biotin-tag at the 5' end and three bases overlapping with the signature sequence; however, this overlapping sequence does not comprise any bases which are specific to the signature sequence (compare first three bases of all *P1for* primers depicted in figure 1, which are identical). Finally, the second forward primer comprises a light-inducible cleavage site, indicated by the "L" in Figure 2.

All of the primers mentioned so far are present in the PCR reaction and said reaction is performed under standard conditions: Initial denaturation at 95°C / 15 min, 40 cycles each comprising the following three steps: 92°C / 45 s; 55°C / 45 s; and 72°C, 45 s comprising the use of a hot start enzyme with buffers according to the manufacturer of the polymerase, and primer concentrations of 0.2 □M.

In should be pointed out that the skilled person knows how to adapt the PCR conditions such that sequence elongation using a primer will only occur if the very last base at the 3' position of the primer anneals to the genomic DNA (e.g. use of Pfu-Polymerase, optionally including mismatches to the sequence in the primers which are hybridising to the template and so on).

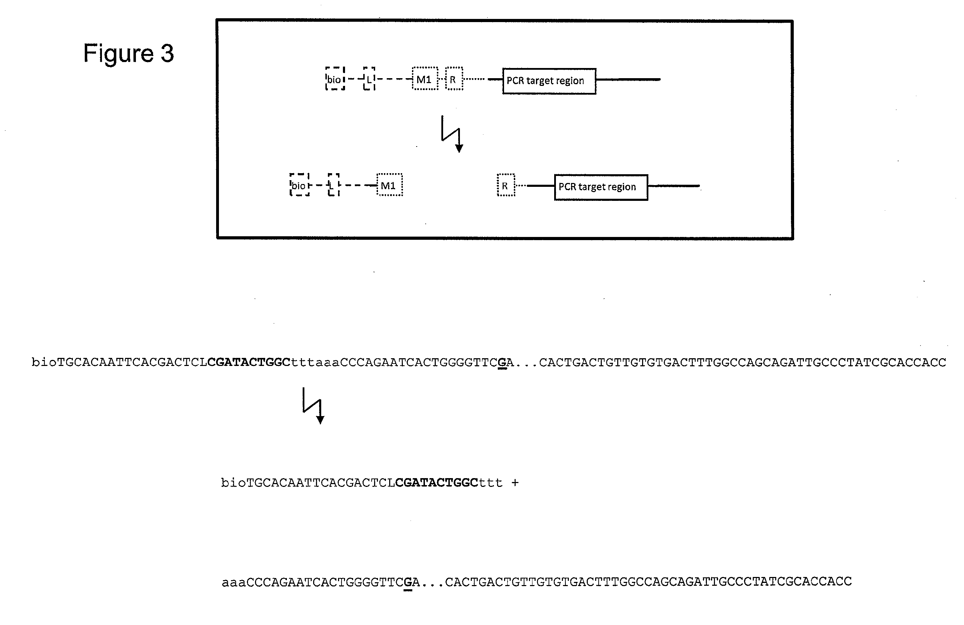

With two identical alleles carrying a G at rs708272, there is only one final product, the sequence of which is shown in figure 3.

Figure 3 also shows the first cleavage step: The resulting PCR fragments are digested with the restriction endonuclease DraI under standard conditions (37°C, NEB buffer 4, 1h, followed by a heat inactivation at 65° / 20 min). This results in a cut after the first three bases of the palindromic recognition site (after the TTT) and, thus, at least two fragments are present in the sample. If there are additional DraI recognition sites, there may be more than two cleavage products present; however, said further products can only arise from the non-biotinylated sequence shown on the bottom since there is no second DraI recognition site present in primer *P2for.*

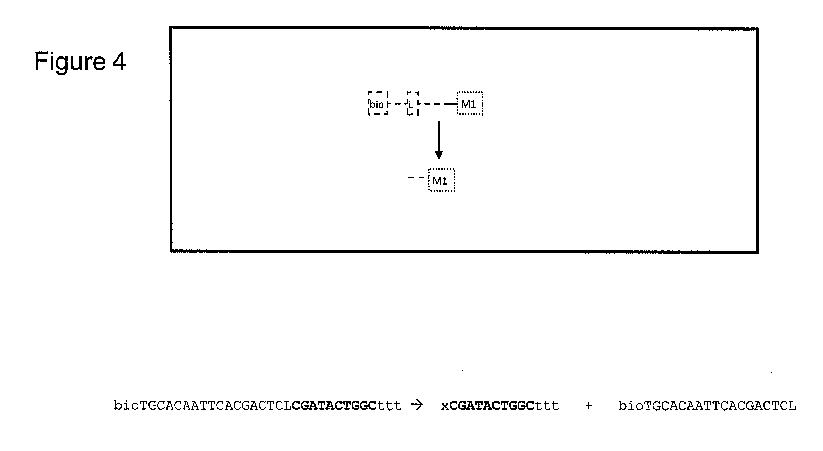

The next two steps shown in Figure 4 relate to a purification and enrichment step, namely the coupling of the biotinylated DNA fragments to a streptavidin matrix in order to enrich the fragments. In the present example, this is carried out according to the GenoSNIP method as described in the manual. Briefly, biotinylated fragments are bound to streptavidin coated wells for at least 20 min in binding buffer BB followed by two wash steps with multiple washes with buffers WP1 and WP2. Subsequently, the enriched fragments are cleaved by UV-irradiation at cleavage site L and eluted in provided pure water. This second cleavage reaction results in a DNA fragment which comprises the unique signature sequence, in the present example CGATACTGGC. In addition to said sequence, three Ts are also present as result of the DraI digestion and a remaining residue X derived from the photocleavable linker.

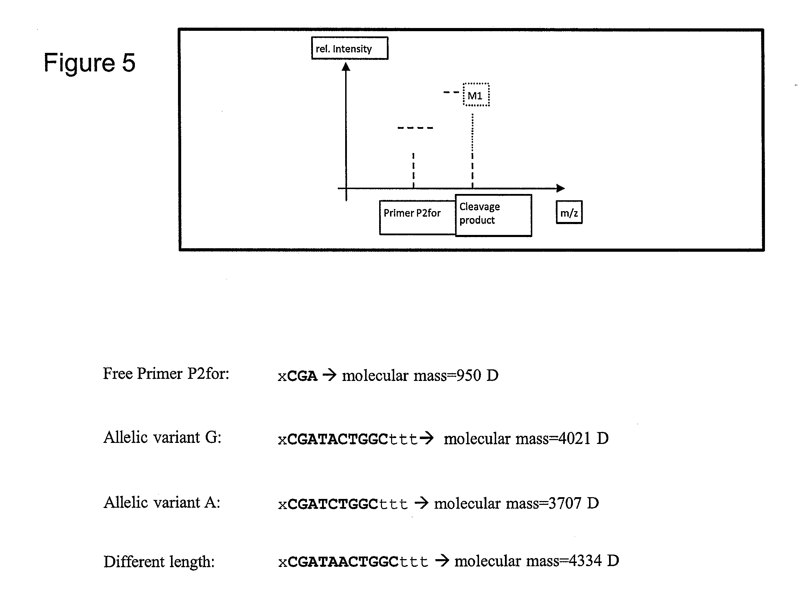

Finally, in the last step, the soluble cleavage products are analysed via mass spectrometry, in this case by regular MALDI-TOF (see figure 5). The expected molecular weights of all possible cleavage fragments (each carrying specific signature sequences) are known from the beginning and, thus, the identification of the corresponding fragment is unambiguous. The only other product which may be detected is primer *P2for* which has not been reacted; however, said primer does not comprise the specific signature and may thus be easily discriminated from respective cleavage products. In the present example, the presence of non-reacted primer would result in a fragment comprising 950 Dalton, whereas the presence of the allelic variant G at rs708272 would result in a fragment of a molecular mass of 4021 Dalton. For variant A at rs708272, a fragment of the molecular weight of 3707 D would be detected.
If the DNA template is comprised of both variants (i.e. if the subject was heterozygous), both first forward primers would have primed corresponding PCR reactions resulting in signals for molecular masses of 4021 D and 3707 D, which can easily be discriminated and assigned to the respective products. In the present example, residue x remaining after the cleavage using UV-light adds a mass of 80 D to each of the fragments.

### Example 2: Detection of a PCR product of a specific length

As shown in Figure 1, a primer *P1for - specific for different DNA length* may also be used according to present setup, e.g. together with primer *P1for - specific for DNA variant G.* A hypothetical ratio for this may be that the region 3' of the underlined G as shown in figure 1 is an intronic region, the presence of which is subject to analysis. Thus, in hypothetical example 2, the position of the underlined G does not represent a polymorphism.

Using a primer covering sequence 5' to the underlined G, one can expect a PCR product of a certain length. Said reaction may represent a control reaction for the overall PCR reaction. However, with the second first forward primer also present in the same PCR reaction, a PCR product can be expected only if said hypothetical intronic sequence is present. In this case, the resulting amplified sequence will be shorter than the sequence amplified using *P1for - specific for DNA variant G.*

As depicted in figure 1, primer *P1for - specific for different DNA length* comprises in 5' to 3' direction the recognition element for the second forward primer followed by a specific signature sequence (in bold) different from the other signature sequence (an additional A is present) and a cleavable site (in small letters), which corresponds to the sequence recognised by the restriction endonuclease DraI, i.e. TTTAAA. At the 3' end, said primer comprises the region which is specific for the intronic DNA template.

The first reverse primer and the second primer pair may be identical to the primers as set out above. All steps are carried out as described above. A cleavage fragment of 4334 D will then indicate that a PCR product was obtained using primer *P1for - specific for different DNA length* (see figure 5 for corresponding fragment). A cleavage fragment of 4021 D will also be identified representing the control reaction.

## Claims

1. Method of detecting at least one specific PCR product comprising at least the steps of:
a) providing a DNA template;
b) providing at least one forward primer wherein each forward primer comprises the following sequence elements in 5' to 3' direction:
a. a specific signature sequence;
b. a cleavable site B;
c. a specific sequence hybridising to the DNA template at the 5' end of the sequence to be amplified;
c) providing at least one reverse primer wherein each reverse primer comprises in 5' to 3' direction a specific sequence hybridising to the DNA template at the 3' end of the sequence to be amplified;
d) performing a PCR reaction using the DNA template of step a) and the primers of steps b) and c);
e) cleaving DNA at said cleavable site to provide DNA cleavage products;
f) analysing DNA cleavage products for the presence of cleavage products comprising said at least one specific signature sequence and thus for the presence of the at least one specific PCR product.

2. Method according to claim 1 comprising at least the steps of:
a) providing a DNA template;
b) providing at least one forward primer wherein each forward primer comprises the following sequence elements in 5' to 3' direction:
a. a cleavable site A;
b. a specific signature sequence;
c. a cleavable site B;
d. a specific sequence hybridising to the DNA template at the 5' end of the sequence to be amplified;
c) providing at least one reverse primer wherein each reverse primer comprises in 5' to 3' direction a specific sequence hybridising to the DNA template at the 3' end of the sequence to be amplified;
d) performing a PCR reaction using the DNA template of step a) and the primers of steps b) and c);
e) cleaving DNA at said cleavable sites to provide DNA cleavage products;
f) analysing DNA cleavage products for the presence of cleavage products comprising said at least one specific signature sequence and thus for the presence of the at least one specific PCR product.

3. Method of detecting at least one specific PCR product comprising at least the steps of:
a) providing a DNA template;
b) providing at least one first forward primer wherein each first forward primer comprises the following sequence elements in 5' to 3' direction:
a. a recognition element for a second forward primer;
b. a specific signature sequence;
c. a cleavable site B;
d. a specific sequence hybridising to the DNA template at the 5' end of the sequence to be amplified;
c) providing at least one first reverse primer wherein each first reverse primer comprises in 5' to 3' direction:
a. a recognition element for a second reverse primer;
b. a specific sequence hybridising to the DNA template at the 3' end of the sequence to be amplified;
d) providing a second forward primer comprising in 5' to 3' direction said recognition element for the second forward primer and optionally a part of said signature sequence;
e) providing a second reverse primer comprising in 5' to 3' direction said recognition element for the second reverse primer;
f) performing a PCR reaction using the DNA template of step a) and the primers of steps b) to e);
g) cleaving DNA at said cleavable site to provide DNA cleavage products;
h) analysing DNA cleavage products for the presence of cleavage products comprising said at least one specific signature sequence and thus for the presence of the at least one specific PCR product.

4. Method according claim 3 comprising at least the steps of:
a) providing a DNA template;
b) providing at least one first forward primer wherein each first forward primer comprises the following sequence elements in 5' to 3' direction:
a. a recognition element for a second forward primer;
b. a specific signature sequence;
c. a cleavable site B;
d. a specific sequence hybridising to the DNA template at the 5' end of the sequence to be amplified;
c) providing at least one first reverse primer wherein each first reverse primer comprises in 5' to 3' direction:
a. a recognition element for a second reverse primer;
b. a specific sequence hybridising to the DNA template at the 3' end of the sequence to be amplified;
d) providing a second forward primer comprising in 5' to 3' direction said recognition element for the second forward primer and optionally a part of said signature sequence wherein a cleavable site A is comprised in said recognition element or 5' to the recognition element;
e) providing a second reverse primer comprising in 5' to 3' direction said recognition element for the second reverse primer;
f) performing a PCR reaction using the DNA template of step a) and the primers of steps b) to e);
g) cleaving DNA at said cleavable sites to provide DNA cleavage products;
h) analysing DNA cleavage products for the presence of cleavage products comprising said at least one specific signature sequence and thus for the presence of the at least one specific PCR product.

5. Method according to any of the preceding claims wherein the analysis of the DNA cleavage products is done by methods selected from the group of methods comprising mass spectrometry, gel elecrophoresis and gel filtration.

6. Method according to any of the preceding claims wherein the DNA cleavage products are purified prior to the analysis by methods selected from the group of methods comprising gel filtration, DNA precipitation, desalting and chromatography with affinity chromatography being preferred.

7. Method according to any of the preceding claims wherein the sequence hybridising to the DNA template and/or the recognition element for the second forward primer and/or the recognition element for the second reverse primer comprises between 10 and 30 nucleotides with 20 nucleotides being preferred.

8. Method according to any of the preceding claims wherein the cleavable site A and/or the cleavable site B are cleavable by methods selected from the group of methods comprising a digestion with restriction endonucleases, UV light irradiation of photocleavable linkers and/or wherein the DNA is cleaved in steps e) and g), respectively, by physical and/or chemical cleavage methods which cleave at unspecific sites within the amplified sequence in such a way that the sizes of the resulting DNA fragments are specific and can be predicted.

9. Method according to claims 2 and 4 wherein the cleavable site A and the cleavable site B are recognition sites for restriction endonucleases and cleavable by a digestion with restrictions endonucleases.

10. Method according to claim 9 wherein the cleavable site A and the cleavable site B are identical and cleavable by a digestion with one restriction endonuclease, preferably with DraI.

11. Method according to claim 9 wherein the cleavable site A and the cleavable site B are different and are cleavable by a digestion with two different restriction endonucleases, preferably with FseI and PmeI.

12. Method according to claims 2 and 4 wherein the cleavable site A comprises a photocleavable linker and is cleavable by UV light irradiation and the cleavage site B is a recognition site for a restriction endonuclease and cleavable by a digestion with a restriction endonuclease, preferably with DraI.

13. Method according to any of the preceding claims wherein the at least one forward primer or the second forward primer is coupled at its 5' end to either one tag of a tag pair selected from the group of tag pairs comprising streptavidin/biotin, hapten/anti-hapten, pairs of complementary nucleotide sequences, aptamers/aptamer targets, histidine glutathione-S-transferase/glutathione, 6X Histidine Tag/Ni-NTA, S-protein/S-peptide, Cutinase/phosphonate inhibitor, antigen/antibody, folic acid/folate binding protein, and protein A or G/immunoglobulins.

14. Method according to claim 13 wherein the at least one forward primer or the second forward primer is coupled at its 5' end to biotin.

15. Method according to claims 12 and 13 wherein the DNA is cleaved by a digestion with a restriction endonuclease at the cleavable site B, preferably with DraI, followed by purification of the tagged DNA and cleavage of said tagged DNA at the cleavable site A by UV light irradiation.

16. Method according to claim 15 wherein the tagged DNA is biotin-tagged and purified via coupling to a streptavidin matrix followed by washing steps.

17. Use of a method according to claims 1 to 16 for detecting at least one specific PCR product in a sample.


SEQUENCE LISTING

<110>  Fraunhofer-Gesellschaft zur Förderung der angewandten
       Forschung e.V.

<120>  Method of reducing the molecular weight of at least one PCR
       product for its detection while maintaining its identity

<130>  F 8692 / DB

<160>  20

<170>  PatentIn version 3.3

<210>  1
<211>  42
<212>  DNA
<213>  Homo sapiens


<220>
<221>  misc_feature
<223>  genomic DNA; chromosome 16: 55,553,755-55,553,796

<400>  1
tgtgtttgtc tgagacccag aatcactggg gttcgagtta gg                    42


<210>  2
<211>  27
<212>  DNA
<213>  Homo sapiens


<220>
<221>  misc_feature
<223>  genomic DNA; chromosome 16: 55,553,929-55,553,955

<400>  2
cactgactgt tgtgtgactt tggccag                                     27


<210>  3
<211>  52
<212>  DNA
<213>  Artificial

<220>
<223>  Primer P1 (specific for DNA variant  A)

<400>  3
gcacaattca cgactcacga tctggcttta aacccagaat cactggggtt ca         52


<210>  4
<211>  53
<212>  DNA
<213>  Artificial

<220>
<223>  Primer P1 (specific for DNA variant  G)

<400>  4
gcacaattca cgactcacga tactggcttt aaacccagaa tcactggggt tcg        53


<210>  5
<211>  54
<212>  DNA

<213>  Artificial

<220>
<223>  Primer P1 (specific for different DNA length)

<400>  5
gcacaattca cgactcacga taactggctt taaatcactg gggttcgagt tagg          54


<210>  6
<211>  42
<212>  DNA
<213>  Artificial

<220>
<223>  Primer P1rev (incl. optional tag for P2rev)

<400>  6
acaacacact gaaaccggtc gtctaacggg atagcgtggt gg                       42


<210>  7
<211>  20
<212>  DNA
<213>  Artificial

<220>
<223>  Primer P2for


<220>
<221>  misc_feature
<222>  (1)..(1)
<223>  biotinylated guanine

<220>
<221>  misc_feature
<222>  (17)..(17)
<223>  light inducible cleavage site

<400>  7
gcacaattca cgactcncga                                                20


<210>  8
<211>  22
<212>  DNA
<213>  Artificial

<220>
<223>  Primer P2rev

<400>  8
gtctaacggg atagcgtggt gg                                             22


<210>  9
<211>  55
<212>  DNA
<213>  Artificial

<220>
<223>  modified genomic DNA (Homo sapiens)

<400>  9
tgcacaattc acgactcacg atactggctt taaacccaga atcactgggg ttcga         55


<210>  10

<211>  49
<212>  DNA
<213>  Artificial

<220>
<223>  modified genomic DNA (Homo sapiens)

<400>  10
cactgactgt tgtgtgactt tggccagcag attgccctat cgcaccacc                49


<210>  11
<211>  55
<212>  DNA
<213>  Artificial

<220>
<223>  modified genomic DNA (Homo sapiens)


<220>
<221>  misc_feature
<222>  (1)..(1)
<223>  biotinylated thymine

<220>
<221>  misc_feature
<222>  (18)..(18)
<223>  light inducible cleavage site

<400>  11
tgcacaattc acgactcncg atactggctt taaacccaga atcactgggg ttcga         55


<210>  12
<211>  49
<212>  DNA
<213>  Artificial

<220>
<223>  modified genomic DNA (Homo sapiens)

<400>  12
cactgactgt tgtgtgactt tggccagcag attgccctat cgcaccacc                49


<210>  13
<211>  31
<212>  DNA
<213>  Artificial

<220>
<223>  modified genomic DNA (Homo sapiens)


<220>
<221>  misc_feature
<222>  (1)..(1)
<223>  biotinylated thymine

<220>
<221>  misc_feature
<222>  (18)..(18)
<223>  light inducible cleavage site

<400>  13
tgcacaattc acgactcncg atactggctt t                                   31

<210>  14
<211>  24
<212>  DNA
<213>  Artificial

<220>
<223>  modified genomic DNA (Homo sapiens)

<400>  14
aaacccagaa tcactggggt tcga                                              24


<210>  15
<211>  49
<212>  DNA
<213>  Artificial

<220>
<223>  modified genomic DNA (Homo sapiens)

<400>  15
cactgactgt tgtgtgactt tggccagcag attgccctat cgcaccacc                   49


<210>  16
<211>  14
<212>  DNA
<213>  Artificial

<220>
<223>  modified genomic DNA (Homo sapiens)


<220>
<221>  misc_feature
<222>  (1)..(1)
<223>  residual moiety after cleavage

<400>  16
ncgatactgg cttt                                                         14


<210>  17
<211>  18
<212>  DNA
<213>  Artificial

<220>
<223>  modified genomic DNA (Homo sapiens)


<220>
<221>  misc_feature
<222>  (1)..(1)
<223>  biotinylated thymine

<220>
<221>  misc_feature
<222>  (18)..(18)
<223>  residual moiety after cleavage

<400>  17
tgcacaattc acgactcn                                                     18


<210>  18
<211>  14
<212>  DNA
<213>  Artificial

<220>
<223>  Allelic variant G


<220>
<221>  misc_feature
<222>  (1)..(1)
<223>  residual moiety after cleavage

<400>  18
ncgatactgg cttt                                                         14


<210>  19
<211>  13
<212>  DNA
<213>  Artificial

<220>
<223>  Allelic variant A


<220>
<221>  misc_feature
<222>  (1)..(1)
<223>  residual moiety after cleavage

<400>  19
ncgatctggc ttt                                                          13


<210>  20
<211>  15
<212>  DNA
<213>  Artificial

<220>
<223>  different DNA length


<220>
<221>  misc_feature
<222>  (1)..(1)
<223>  residual moiety after cleavage

<400>  20
ncgataactg gcttt                                                        15